# EUROPÄISCHE PATENTSCHRIFT

(11) **EP 0 971 771 B1**
(45) Veröffentlichungstag und Bekanntmachung des Hinweises auf die Patenterteilung: **04.12.2002**
(21) Anmeldenummer: 99907434.7
(22) Anmeldetag: 27.01.1999
(51) Int. Cl.: A61N 2/02

(54) **MAGNETFELDDECKE**
MAGNETIC FIELD BLANKET
COUVERTURE A CHAMP MAGNETIQUE

(30) Priorität: 29.01.1998 DE 19803428
(43) Veröffentlichungstag der Anmeldung: 19.01.2000
(73) Patentinhaber: Hasse, Erwin, 89407 Dillingen (DE)
(72) Erfinder: SAWICKI, Eduard, D-86639 München (DE); HASSE, Mariola, D-89407 Dillingen (DE); HASSE, Ines, D-89407 Dillingen (DE)
(74) Vertreter: Barske, Heiko, Dr. rer. nat.
(86) Internationale Anmeldenummer: EP9900527
(87) Internationale Veröffentlichungsnummer: WO99038570

(56) Entgegenhaltungen:
- EP-A- 0 239 098
- DE-A- 3 205 048
- FR-A- 2 582 521
- US-A- 4 266 533
- US-A- 4 993 413
- US-A- 5 084 003
- US-A- 5 743 844

## Beschreibung

Die Erfindung betrifft eine Magnetfelddecke gemäß dem Oberbegriff des Hauptanspruchs.

Magnetfelddecken werden für unterschiedlichste therapeutische Zwecke eingesetzt.

Eine gattungsgemäße Magnetfelddecke ist aus der EP 0 239 098 B1 bekannt. Diese Magnetfelddecke weist wenigstens eine Magnetfeldspule auf, deren Leitungsdraht sich mäanderförmig über einen großflächigen Bereich der Magnetdecke erstreckt. Das Verlegen des Leitungsdrahtes einer solchen Spule ist verhältnismäßig aufwendig. Außerdem sind zum Erzeugen von Magnetfeldern einer vorbestimmten Stärke innerhalb der Spule verhältnismäßig große Stromstärken erforderlich, da sich die Magnetfelder der einzelnen zueinander parallelen Strompfade teilweise aufheben.

In der DE 32 05 048 A ist ein Flachbandkabel beschrieben, das schraubenförmig um ein Pferdebein gelegt wird, wobei die voneinander beabstandeten Enden des Flachbandkabels mit Anschlüssen derart verbunden sind, daß sie teilweise zueinander parallel und teilweise in Reihe geschaltet sind. Weiter zeigt diese Druckschrift eine Manschette mit integrierten Magnetspulen, wobei die Magnetspulen durch spiralförmig angeordnete Adern gebildet sind.

Die US 4,993,413 zeigt, ähnlich wie die vorgenannte Druckschrift, ein Flachbandkabel, das um ein Bein schraubenförmig herumgewickelt wird, wobei die an den Enden des Flachbandkabels freiliegenden Adern derart miteinander verbunden sind, daß die Adern in Reihe geschaltet sind.

Der Erfindung liegt die Aufgabe zugrunde, eine Magnetfelddecke zu schaffen, die bei kostengünstiger Herstellbarkeit einen guten Wirkungsgrad hinsichtlich der Umsetzung der zugeführten elektrischen Leistung in das therapeutisch wirksame Magnetfeld aufweist. Vorteilhafterweise soll dieses Magnetfeld gezielt an die jeweiligen Bedürfnisse anpaßbar sein.

Der erste Teil der Erfindungsaufgabe wird mit den Merkmalen des Hauptanspruchs gelöst. Mit dem erfindungsgemäß vorgesehenen Flachbandkabel kann in außerordentlich kostengünstiger Weise eine großflächige Magnetfeldspule mit einer der Anzahl der Leiter des Flachbandkabels entsprechenden Windungszahl hergestellt werden. Innerhalb einer solchen Magnetfeldspule herrscht ein weitgehend homogenes Magnetfeld, dessen Stärke von der Windungszahl und der Stromstärke abhängt.

Die Unteransprüche 2 und 3 sind auf eine vorteilhafte Kontaktierung der Adern des Flachbandkabels gerichtet.

Die Unteransprüche 4 bis 7 sind auf Ausführungsformen der Magnetfelddecke gerichtet, bei der sich durch gezielte Ansteuerung der einzelnen Spulen von einer Stromimpulsgebereinheit aus das Magnetfeld innerhalb der großflächigen Magnetfeldspule an den jeweiligen therapeutischen Bedarf anpassen läßt.

Die Erfindung wird im folgenden anhand schematischer Zeichnungen beispielsweise und mit weiteren Einzelheiten erläutert.

Es stellen dar:
- Fig. 1: eine Aufsicht auf eine Magnetfelddecke und
- Fig. 2: eine Aufsicht auf eine Anschlußeinheit für die einzelnen Spulen.

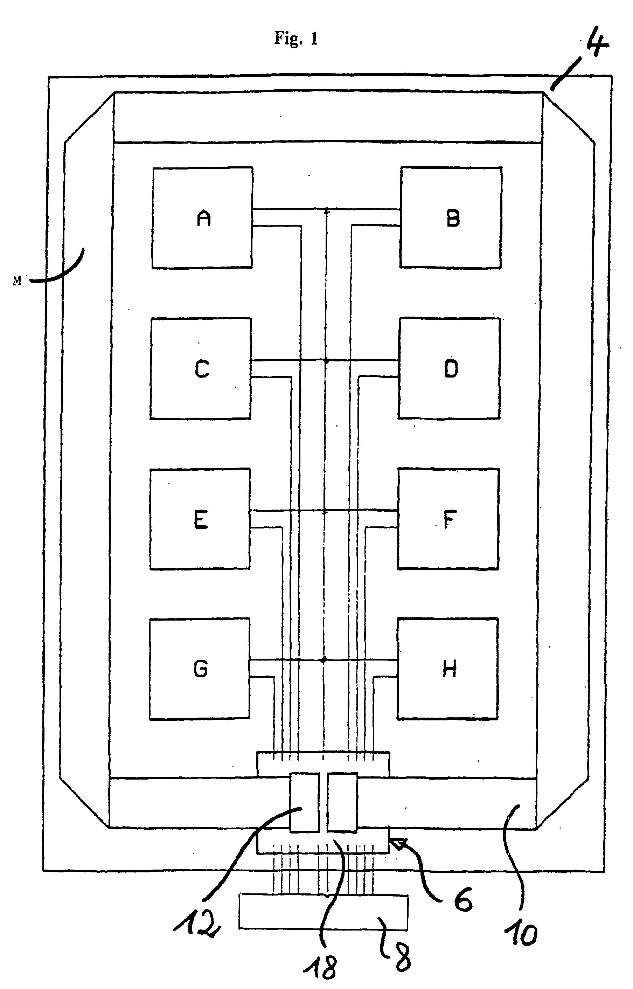

Gemäß Fig. 1 sind in eine Magnetfelddecke 4 eine Magnetfeldspule M und mehrere Zusatzspulen A bis H integriert. Die Spulen sind über eine Anschlußeinheit 6 mit einem Stromimpulsgeber 8 verbunden.

Der Aufbau der Magnetfelddecke kann in an sich bekannter Art sein, beispielsweise mit zwei aus Stoff bestehenden Deckschichten, die zwischen sich eine beispielsweise zweilagige Polsterschicht aufnehmen, zwischen deren Lagen die Spulen und die Anschlußeinheit 6 angeordnet sind.

Die Magnetfeldspule M, die in der Nähe des Umfangs der Magnetfelddecke 4 verläuft, ist durch ein Flachbandkabel 10 gebildet, das in den Ecken der Decke umgefaltet ist und somit insgesamt flach angeordnet ist. Das Flachbandkabel kann handelsüblicher Bauart mit einer an den jeweiligen Bedarf angepaßten Anzahl von Adern sein. Die Enden des Flachbandkabels liegen sich im Bereich der Anschlußeinheit gegenüber und sind auf weiter unten beschriebene Weise kontaktiert.

Die Zusatzspulen A bis H sind in zwei nebeneinanderliegenden Reihen innerhalb der insgesamt rechteckigen Magnetfeldpsule M angeordnet. Jeweils ein Anschluß der Zusatzspulen ist mit einer gemeinsamen Masseleitung verbunden, an die auch ein Anschluß der Magnetfeldspule M gelegt sein kann. Die anderen Anschlüsse sind getrennt aus der Anschlußeinheit 6 heraus geführt und mit dem Stromimpulsgeber 8 verbunden.

Der Stromimpulsgeber 8 ist beispielsweise aufgebaut wie in der eingangs genannten Druckschrift beschrieben und wird daher nicht erläutert. Mit dem Stromimpulsgeber 8 können die einzelnen Spulen individuell hinsichtlich Frequenz, Form und Stärke bzw. Amplitude der einzelnen Stromimpulse angesteuert werden, so daß das in den einzelnen Bereichen der Magnetfelddecke herrschende Magnetfeld individuell beeinflußt und an die jeweiligen therapeutischen Zwecke angepaßt werden kann.

Die Zusatzspulen A bis H sind vorteilhafterweise ähnlich wie die Magnetfeldspule M ebenfalls durch Flachbandkabel gebildet.

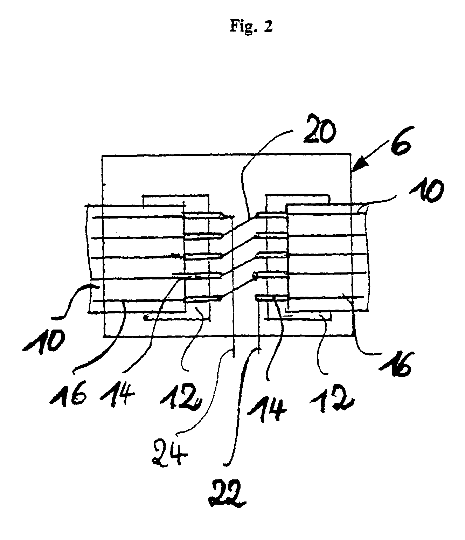

Gemäß Fig. 2 sind die Enden des Flachbandkabels 10 beispielsweise in an sich bekannten sogenannten DIL-Steckern 12 aufgenommen, deren Kontaktzahl auf die Anzahl der Adern des Flachbandkabels abgestimmt ist. Die Stecker weisen ein Unterteil auf, in dem die in nach oben vorstehenden Messern endenden Kontakte 14 gehalten sind, und ein Oberteil. Ein Ende des Flachbandkabels wird auf das Unterteil derart aufgelegt, daß sich je eine Ader 16 des Flachbandkabels 10 über einem der Kontakte 14 befindet und beim Verbinden des Oberteils des Steckers mit dem Unterteil durch die Isolierung des Flachbandkabels hindurch von dem Kontakt kontaktiert wird.

Die Kontakte 14 der Stecker 12 sind durch Stifte gebildet, die nach hinten abgebogen sind, und in Löcher einer Leiterplatte 18 eingesteckt sind, die mit Leiterbahnen 20 versehen ist. Die Verbindung der Stecker 12 mit den Leiterbahnen 20 erfolgt beispielsweise durch Löten.

Die Leiterbahnen 20 sind derart ausgebildet, daß das Ende einer ersten Ader 16 des Flachbandkabels 10 mit einem Anschluß 22 verbunden ist, der beispielsweise der allen Spulen gemeinsame Masseanschluß ist. Das andere Ende dieser ersten Ader ist mit dem Anfang einer zweiten Ader verbunden, deren Ende wiederum mit dem Anfang der nächsten Ader verbunden ist usw. bis schließlich das Ende der letzten Ader zu dem Anschluß 24 herausgeführt ist. Auf diese Weise bildet das Flachbandkabel 10 die Magnetfeldspule M mit einer der Anzahl seiner Adern entsprechenden Windungszahl. Der Einfachheit halber sind in Fig. 2 die den elektrischen Anschlüssen der Zusatzspulen dienenden Leiterbahnen und Anschlüsse nicht eingezeichnet. Die Zusatzspulen selbst weisen, wenn sie als Flachbandkabel ausgebildet sind, ähnliche Anschlußeinheiten auf, die insgesamt flach und mit kleinem Flächenbedarf ausgebildet sind, so daß sie die Handhabbarkeit der in jeder Weise faltbaren Decke nicht beeinträchtigen.

Es versteht sich, daß die beschriebene Decke in vielfacher Hinsicht abgeändert werden kann. Die Form und Anordnung der Zusatzspulen kann abgeändert werden. Die elektrischen Anschlüsse können in abgeänderter Weise erfolgen. Bei vieladrigen Flachbandkabeln (z.B. 100 Adern) können einzelne der Adern parallel geschaltet werden, wenn die hohe Windungszahl nicht benötigt wird. Die Zusatzspulen müssen nicht durch Flachbandkabel gebildet sein. Das Flachbandkabel kann, um die Windungszahl weiter zu erhöhen, zu zwei Schleifen gelegt sein. Die Zusatzspulen müssen nicht alle individuell ansteuerbar sein. Einzelne Zusatzspulen können auch außerhalb der Magnetfeldspule M angeordnet sein. Die Magnetfelddecke kann deutlich größer als die Magnetfeldspule sein, die zum Behandeln großer Flächen geeignet ausgebildet ist. Der Schutzbereich der Erfindung wird durch die Patentansprüche definiert.

## Patentansprüche

1. Magnetfelddecke mit wenigstens einer Magnetfeldspule (M), die in der Decke flach angeordnet und insgesamt rechteckig ist und Anschlüsse (22) zum Anschließen an einen Stromimpulsgeber (8) aufweist, **dadurch gekennzeichnet, daß** die Magnetfeldspule (M) aus einem an den Ecken der Spule umgefalteten Flachbandkabel (10) gebildet ist, dessen Enden sich gegenüberliegend angeordnet sind, und wobei zumindest einige der sich gegenüberliegenden Enden der nebeneinander angeordneten Adern (16) des Flachbandkabels derart miteinander verbunden sind, daß die zugehörigen Adern in Reihe hintereinander geschaltet sind.

2. Magnetfelddecke nach Anspruch 1, **dadurch gekennzeichnet, daß** die Enden der Adern (16) des Flachbandkabels (10) in Steckern (12) kontaktiert sind, deren Kontakte (14) mit Leiterbahnen (20) einer Leiterplatte (18) verbunden sind, die die Verbindungen zwischen den einzelnen Adern und zu äußeren Anschlüssen (22, 24) der Magnetfeldspule (M) enthält.

3. Magnetfelddecke nach Anspruch 2, **dadurch gekennzeichnet, daß** der Anschluß des Flachbandkabels (10) an die Kontakte (14) der Stecker (12) lötfrei und der Kontakte an Leiterbahnen (20) der Leiterplatte (18) mittels Lötung erfolgt.

4. Magnetfelddecke nach einem der Ansprüche 1 bis 3, **dadurch gekennzeichnet, daß** im Inneren der Magnetfeldspule (M) Zusatzspulen (A bis H) angeordnet sind.

5. Magnetfelddecke nach Anspruch 4, **dadurch gekennzeichnet, daß** die Magnetfeldspule (M) die Form eines Rechtecks mit unterschiedlichen Seitenlängen hat und die Zusatzspulen (A bis H) innerhalb des Rechtecks in zwei Reihen angeordnet sind.

6. Magnetfelddecke nach einem der Ansprüche 1 bis 5, **dadurch gekennzeichnet, daß** außerhalb der Magnetfeldspule (M) Zusatzspulen angeordnet sind.

7. Magnetfelddecke nach einem der Ansprüche 4 bis 6, **dadurch gekennzeichnet, daß** alle Zusatzspulen (A bis H) durch Flachbandkabel gebildet sind.

## Claims

1. Magnetic field blanket including at least one magnetic field coil (M), having a generally rectangular shape and said coil being flatly accommodated within said blanket and comprising connections (22) adapted to be connected with a current pulse generator (8), **characterized in that** said magnetic field coil (M) is formed by a flat band cable (10), which is folded at the corners of said coil and the ends of which are located opposite to each other, wherein at least some of the opposite ends of the leads (16) of said flat band cable located side by side are connected with each other such that the corresponding leads are connected in series.

2. Magnetic field blanket according to claim 1, **characterized in that** the ends of the leads (16) of said flat band cable (10) are contacted in connectors (12), the contacts (14) of which are connected to conductors (20) of a printed circuit board (18), which includes the connections between the single leads and to the outer connections (22, 24) of said magnetic coil (M).

3. Magnetic coil blanket according to claim 2, **characterized in that** the connection of said flat band cable (10) to the contacts (14) of the connectors (12) is without soldering and of the contacts to the conductors (20) of the printed circuit board (18) by soldering.

4. Magnetic coil blanket according to one of claims 1 to 3, **characterized in that** additional coils (A to H) are located within ventilate of the magnetic field coil (M).

5. Magnetic field blanket according to claim 4, **characterized in that** said magnetic field coil (M) has the shape of a rectangle with sides of different lengths and said additional coils (A to H) are located within said rectangle in two rows.

6. Magnetic field blanket according to any of claims 1 to 5, **characterized in that** additional coils are located outside of said magnetic field coil (M).

7. Magnetic field blanket according to any of claims 4 to 6, **characterized in that** all additional coils (A to H) are formed by flat band cables.

## Revendications

1. Couverture à champ magnétique comportant au moins une bobine de champ magnétique (M) qui est agencée à plat dans la couverture, qui est dans l'ensemble rectangulaire et qui comprend des bornes (22) pour le raccordement à un générateur d'impulsions de courant (8), **caractérisée en ce que** la bobine de champ magnétique (M) est formée par un câble plat (10) replié aux coins de la bobine, dont les extrémités sont agencées en vis-à-vis l'une de l'autre, et **en ce qu'**au moins quelques-unes des extrémités opposées des conducteurs (16) agencés les uns à côté des autres du câble plat sont reliées les unes aux autres de telle sorte que les conducteurs associés sont branchés en série les uns derrière les autres.

2. Couverture à champ magnétique selon la revendication 1, **caractérisée en ce que** les extrémités des conducteurs (16) du câble plat (10) sont mises en contact dans des prises (12) dont les contacts (14) sont connectés à des pistes conductrices (20) d'une carte imprimée (18) qui comprend les connexions entre les conducteurs individuels et vers des bornes extérieures (22, 24) de la bobine de champ magnétique (M).

3. Couverture à champ magnétique selon la revendication 2, **caractérisée en ce que** le raccordement du câble plat (10) aux contacts (14) des prises (12) s'effectue sans brasage, et celui des contacts aux pistes conductrices (20) de la carte imprimée (18) s'effectue par brasage.

4. Couverture à champ magnétique selon l'une des revendications 1 à 3, **caractérisée en ce que** des bobines supplémentaires (A à H) sont agencées à l'intérieur de la bobine de champ magnétique (M).

5. Couverture à champ magnétique selon la revendication 4, **caractérisée en ce que** la bobine de champ magnétique (M) présente la forme d'un rectangle à longueurs latérales différentes et les bobines supplémentaires (A à H) sont agencées dans deux rangées à l'intérieur du rectangle.

6. Couverture à champ magnétique selon l'une des revendications 1 à 5, **caractérisée en ce que** des bobines supplémentaires sont agencées à l'extérieur de la bobine de champ magnétique (M).

7. Couverture à champ magnétique selon l'une des revendications 4 à 6, **caractérisée en ce que** toutes les bobines supplémentaires (A à H) sont formées par des câbles plats.
